Europäisches Patentamt

European Patent Office

Office européen des brevets

(19)

(11) Publication number: 0 293 606
A2

## (12) EUROPEAN PATENT APPLICATION

(21) Application number: 88106951.2

(22) Date of filing: 29.04.88

(51) Int. Cl.4: C07K 3/18 , C07K 17/00 , C12P 21/00 , C12N 5/00 , C12N 15/00 , //G01N33/68, G01N33/577,G01N33/543

The applicant has filed a statement in accordance with Rule 28 (4) EPC (issue of a sample only to an expert). Accession numbers of the deposits: ATTC HB - 8759, CLR - 1581, HB - 9159 and HB - 9160.

(30) Priority: 01.05.87 US 45350

(43) Date of publication of application:
07.12.88 Bulletin 88/49

(84) Designated Contracting States:
AT BE CH DE ES FR GB GR IT LI LU NL SE

(71) Applicant: Xoma Corporation
2910 Seventh Street
Berkeley California 94710(US)

(72) Inventor: Urnovitz, Howard
554 - 16th Avenue
San Francisco California 94118(US)
Inventor: Eaton, Linda
1059 Bret Knoll Court
San Jose California 95120(US)

(74) Representative: Glawe, Delfs, Moll & Partner
Patentanwälte
Postfach 162 Liebherrstrasse 20
D-8000 München 26(DE)

(54) Immunoglobulin purification by selective anti-idiotype binding.

(57) Purification methods and composition are disclosed for the isolation of a first immunoglobulin. Hybridomas are produced which secrete antibodies directed to an idiotype region of the first immunoglobulin and from these hybridomas, ones are selected which secrete a second immunoglobulin which binds to the first at an idiotopic site under a first environment and which dissociates under a second environment which does not significantly alter the binding characteristics of either immunoglobulin. A support is coupled to the second immunoglobulin and contacted with a sample containing the first immunoglobulin under the first environment. A complex is formed between the anti-idiotope immunoglobulin and the idiotopic site of the first immunoglobulin. Non-complexed components in the sample are separated, the complex is dissociated under the second environment, the first immunoglobulin is isolated to yield a purified product having increased activity.

The hybridoma XMMME-001 was deposited with the American Type Culture Collection (A.T.C.C.) on March 26, 1985, and given A.T.C.C. Accession No. HB8759. The hybridoma XMMME-003 was deposited with the A.T.C.C. on July 3, 1986, and given A.T.C.C. Accession No. HB9159. Hybridoma XMMME-IO1 was deposited with the A.T.C.C. on July 3, 1986, and given A.T.C.C. Accession No. HB9160.

Xerox Copy Centre

## IMMUNOGLOBULIN PURIFICATION BY SELECTIVE ANTI-IDIOTYPE BINDING

BACKGROUND OF THE INVENTION

1. Field of the Invention

As the use of antibodies and antibody derivatives increases in research, medical diagnosis and treatment, and industrial applications, there is an associated increase in the need for purification techniques that yield antibodies that are not only free from foreign antibodies but are also free from essentially identical antibodies that have decreased or altered binding characteristics. Such purification techniques relate generally to immunoadsorption procedures.

2. Description of the Relevant Art

Immunoglobulin (Ig) molecules, including antibodies or fragments thereof, protect a host from infectious agents, such as bacteria, viruses, fungi, parasites or the like, in part by their ability to recognize a wide variety of antigens associated with such agents. Monoclonal antibodies (MoAbs) are homogeneous immunoglobulins resulting from the proliferation of a single clone of antibody-producing cells.

Immunoglobulins contain variable and constant regions in their constituent polypeptide chains. Antigenic binding properties of an antibody are determined by the amino acid sequence of the variable domains of the light and heavy chains of the antibody which together form a combinational variable region of the antigen binding sites. The principal three-dimensional structure is preserved in antibodies. The individual antigen binding characteristics of a particular antibody are attributable to the variable region. Amino acid side chains in this region play a major role in the binding characteristics and affinity of the antibody for its epitope (antigenic determinant). MoAbs directed against a particular antigenic determinant are now routinely produced in high yields by means of hybridoma technology developed by Köhler and Milstein, Nature (1975) 256:495-97. Epitope binding by any one type of monoclonal antibody is described by a set of characteristic binding properties.

Since immunoglobulin molecules themselves are usually antigenic, antibodies can be produced that recognize antigenic determinants in both the constant and variable regions of immunoglobulin molecules. Antigenic determinants in the variable region associated with an antigen binding site of an immunoglobulin molecule are termed idiotopes. A number of idiotopes may be found on any one antibody and collectively make up the idiotype region or idiotype of that antibody.

Anti-idiotype antibodies have been generated experimentally and thereafter studied extensively over the last decade. Idiotype-anti-idiotype interactions are theorized to regulate the immune response (Jerne, Ann. Immunol. Inst. Pasteur (Paris) (1974) 1256:378-89). Anti-idiotype antibodies have been employed as in vivo diagnostic probes (Halpern, et al., J. Immunol. (1984) 133:1852-856). Monoclonal anti-idiotype antibodies have been used to immunize mice against lethal Streptococcus pneumoniae (McNamara, et al., Science (1984) 226 (4680):1325-326). Also, an anti-idiotype monoclonal antibody was successfully employed to attack malignant cells of a non-secretory B cell lymphoma (Miller, et al., NEJM (1982) 306:517-22).

Immunoadsorption procedures that use antibodies or fragments of antibodies linked to a solid support are known in the art. Further. MoAbs have been isolated which bind antigens at one pH while dissociating at a second pH and are described in International Publication No. WO 83/03678. See, for example, Affinity Chromatography and Related Techniques, Proc. of the Fourth International Symposium, Veldhove, The Netherlands, June 22-26, 1981, Ed. P.C.J. Gribman, et al., Elsevier Sci. Pub. Co., N.Y., 1982; U.S. Pat. Nos. 3,957,741; 4,059,685; and International Publication No. WO 83/03678. Further, immunoadsorption techniques using anti-idiotype antibodies coupled to a solid support are currently under investigation. See, Gheuens and McFarlin, Eur. J. Immunol. (1982) 12:701-703 and Australian Application No. AU-A-90193/82.

Immunoglobulins may be altered in a number of ways. Various procedures for fragmenting immunoglobulins are well known in the art. See, Hudson and Hay, Practical Immunology, 2nd ed., Blackwell Scientific Publication, 1980, p, 192. Also, monoclonal antibodies have been chemically modified by a variety of moieties in the prior art (Carlsson, et al., Biochem. J. (1978) 173:723-27). One particularly relevant type of chemical modification involves a monoclonal antibody or a fragment thereof combined with a cytotoxic agent to form a conjugate known as an immunotoxin. See, for example, Gilliland, et al., Proc. Nat'l. Acad. Sci. U.S., (1980) 77:4539-543, Kernan, et al., J. Immunol. (1984) 133(1):137-46, Embleton, et al., Brit. J. Can. (1984) 49(3):559-65, and Hammersmith Oncology Group, Lancet (1984) 1(8392):1441-443.

## SUMMARY OF THE INVENTION

Purification methods and compositions are provided for the isolation of a first immunoglobulin of interest. Hybridomas are produced which secrete immunoglobulin specific for an idiotype region of the first immunoglobulin. A particular hybridoma is selected which secretes a second immunoglobulin which binds to the first immunoglobulin at an idiotopic site in the idiotype region under a first environment and which dissociates under a second environment such that the binding characteristics of the first immunoglobulin, and of the second, are not significantly altered. The second immunoglobulin is coupled to a support and contacted with a sample containing the first immunoglobulin under the second environment, forming a complex with the coupled first immunoglobulin. The complex is then separated from any non-complexed components in the sample. Alternatively, the first immunoglobulin is chemically modified, such as by conjugation with a therapeutic or diagnostic moiety or the like, and then separated from non-complexed or non-conjugated ligand. In this case, molecules of the first immunoglobulin, whose binding properties have been damaged by the conjugation, are separated from those molecules with retained activity. The complex is dissociated under the second environment and the first immunoglobulin or conjugate is isolated in a purified form. The resulting purified immunoglobulins or conjugates find use as therapeutic, diagnostic or analytic reagents.

## BRIEF DESCRIPTION OF THE DRAWINGS

Fig. 1 depicts the inhibition of XMMME 001 antibodies binding to specific target cells by the antibody product of cell line XMMME-IO1, using Fluorescence Activated Cell Sorter (FACS) analysis;

Fig. 2 is a FACS competition assay showing the binding of XMMME-001 antibody (purified using solid support coupled XMMME-IO1 antibody) to specific target cells; and

Fig. 3 depicts a FACS competition assay in which XMMME-001 antibody conjugated to the A-chain of ricin (and purified from a column containing solid support coupled to XMMME-IO1 antibody) is bound to specific target cells.

## DETAILED DESCRIPTION OF THE SPECIFIC EMBODIMENTS

The present invention provides those skilled in the art with a method of purifying immunoglobulins (Ig) molecules, including antibodies, fragments of antibodies, and antibody derivatives produced through chemical modification. Immunoadsorption techniques are employed to isolate an immunoglobulin of interest and represent a significant improvement over the prior art methods. Generally, anti-idiotype antibodies are selected that bind under one set of mild conditions at the idiotype region of an antibody of interest and release under a second set of mild conditions such that the binding characteristics of the immunoglobulins are not significantly altered, thereby allowing for the identification and isolation of the antibody of interest.

In particular, the purification method for isolating a first immunoglobulin comprises: (a) producing a plurality of hybridomas, each of which secretes immunoglobulins specific for an idiotype region of the first immunoglobulin; (b) selecting from among the hybridomas a hybridoma secreting a second immunoglobulin which binds to the first immunoglobulin at an idiotopic site in the idiotype region under a first environment and dissociates from the first immunoglobulin under a second environment; (c) coupling the second immunoglobulin to a support; (d) contacting in an aqueous medium a sample containing the first immunoglobulin with the support-coupled second immunoglobulin under the first environment, thereby forming a complex between the first and the second immunoglobulins; (e) separating the complex from any non-complexed components in the sample or chemically modifying the first immunoglobulin, such as by conjugation with a therapeutic or diagnostic moiety or the like, prior to such separation; (f) dissociating the complex under the second environment; and (g) collecting the first antibody, fragment thereof, or derivative thereof in a purified form.

Hybridomas secreting monoclonal antibodies (MoAbs) are produced according to the now classic techniques developed by Köhler and Milstein. Although mouse myeloma cells are employed as an example of the fusion partner for mouse spleen cells extracted from immunized mice, other immunoglobulin producing cells from mammals such as rat or human may be employed. See, e.g. Terg, et al., Proc. Nat'l. Acad. Sci. U.S. (1985) 82:1790-94. The fusion partner is not critical to the present invention and any such partners resulting in immortal cells secreting anti-idiotype monoclonal antibodies may be employed. Although hybridoma technology is described in some detail as a way to produce monoclonal antibodies, any of the currently known or future methods for monoclonal antibody production are contemplated in this disclosure. Cell line immortalization may be by fusion, transformation or other similar procedure.

Immunoglobulin class (IgG, IgA, and the like) or subclass ($IgG_1$, $IgG_2$, and the like) molecules are contemplated as acceptable and appropriate species to be employed in connection with the present invention. Fragments of immunoglobulins may also be employed as the first or second immunoglobulin. Those skilled in the relevant art are well versed in the production of various antibody fragments (Fab, F(ab')₂, and the like). See, Hudson and Hay, Practical Immunology, 2nd ed., Blackwell Scientific Publications, 1980, p. 192 and Watson, Molecular Biology of the Gene, 3rd ed., Benjamin/Cummings, Menlo Park, Calif., 1977, p. 605.

After a plurality of hybridomas which secrete anti-idiotype MoAbs are produced, particular hybridomas are selected which secrete an antibody, termed "second" immunoglobulin, which binds to the immunoglobulin of interest, termed "first" immunoglobulin, under one set of conditions, termed "first" environment, and dissociates under a different set of conditions, termed "second" environment. Included in these conditions or environments would be such variables as pH, ionic strength, presence and concentration of chaotropic agents, temperature and the like. Both the first and second environments must be such that no significant alterations occur to the antigen binding characteristics of either the first or second immunoglobulins. This implies that "mild" conditions be employed so as not be denature or partially denature the concerned immunoglobulin structure beyond a readily reversible form and that any slight alterations in antigen binding constants will not be such as to interfere with native-like binding behavior by the immunoglobulin.

Usually, the determined difference between the first environment and second environment will be a change in pH or ionic strength of the aqueous solution. More usually, the difference will be a change in pH from a first environment of about pH 6-10 to a second environment of about pH 3-7. Preferred is a first environment pH of about pH 6.5-8.5 and a second environment pH of about pH 4-6 and more preferred is a first environment pH of about pH 8-8.5 and a second environment pH of about pH 4.5-5. As stated above, it is contemplated that in addition to altering the pH values between the first and second environments, other solution variables may also be changed such as ionic strength or ionic species.

In the past, the selection process for choosing hybridomas which secrete anti-idiotope MoAbs which dissociate from the immunoglobulin molecule of interest was a two-step process and may have required considerable experimentation, however, by using enzyme immunoassays (EIA) employing a Hybri-dot manifold (Bethesda Labs), or similar device, in conjunction with the modifications described herein, the selection of suitable hybridomas require only several days of laboratory work rather than several weeks or even months. For example, following the selection of one or more hybridomas secreting anti-idiotope MoAbs, further selection for those hybridomas secreting anti-idiotope MoAbs which dissociate at say about pH 5 can be readily accomplished. As above, although pH is the exemplified environmental condition, temperature, ionic strength, and the like may be assayed solely or in combination as well.

Coupling of the second immunoglobulin to a support may be accomplished by any one of numerous means well known in the art. See, Methods in Enzymology: Immortalized Enzymes, (1976) 44, Ed. Mosbach, Academic Press. The support-coupled second immunoglobulin is employed to temporarily bind the first immunoglobulin under the first environment forming a complex so that any contaminating, non-complexing components in the sample containing the first immunoglobulin may be separated from the complex. Separation may be by methods such as column chromatography, batch procedures and the like. The support may be a solid support such as a particle (e.g. bead, fiber, or the like) or container wall and would include materials such as nitrocellulose, cellulose, agarose, polystyrene, dextran, glass, polymer coated magnetic metals, nylon, silica gel, polyacrylamide, polymethyl methacrylate, and the like. It is well within the skill of the art to select a support suitable for the reactive first and second environments of choice, and to bind the immunoglobulin to the support.

The second immunoglobulin may be coupled directly to the support or via a spacer arm to aid in preventing any possible charge or steric interferences with antigen binding. Various spacer arms are available and well known in the art. The molar ratio of second immunoglobulin to active coupling sites on the support may be about 1:25, usually about 1:100 to 1:200, and may be 1:400 or lower.

Usually, the sample containing the antibody of interest (first antibody, fragment thereof, or derivative thereof) will be in an aqueous medium. The coupled second immunoglobulin will be contacted with the sample so as to form a complex under the first environment. The complex is between the second immunoglobulin and the idiotype region of the first immunoglobulin. Non-complexed components (ones not bound by the second immunoglobulin) in the sample may be separated from the bound first immunoglobulin by any technique appropriate to the support employed, including, but not limited to, chromatography, electrophoresis, centrifugation, magnetic separation, filtration and the like.

After the support-coupled complex is separated from the non-complexing components, the first environment is altered so as to produce the second environment thereby causing the complex to dissociate. As stated above, this change in conditions may reflect pH, ionic strength or similar variations. The requirement exists that the high affinity for an idiotope of the first immunoglobulin by the second immunoglobulin under the first environment be changed to produce a low affinity form of the second immunoglobulin so the complex dissociates. The alteration in environment should be such that the binding characteristics of either the first or second immunoglobulins is not significantly altered.

Following the dissociation of the complex, the first immunoglobulin is collected in a purified form by a technique suited to the support employed and includes, but is not limited to, chromatography, electrophoresis, centrifugation, filtration, magnetic separation, and the like.

Purified products produced by the present invention find utility in a variety of diagnostic, therapeutic and analytic applications. The products may be diluted to a required concentration for administration to a host in a vehicle such as sterile water, normal saline, adjuvant, or the like. The product may be administered in vivo to a host by any convenient route such as intramuscularly, intravenously, intraperitoneally, or the like. The purified products may also be administered directly to cell cultures following dilu- tion.

Either the first or second immunoglobulins may be chemically modified to produce a derivative or conjugate. Normally, the first immunoglobulin would be more likely to be chemically modified than the second immunoglobulin, but this does not limit modification to the first alone. Various chemical moieties may be coupled to the immunoglobulin to produce conjugates. Such moieties may be, but are not limited to, enzymes, chromaphores, nuclides, and cofactors), therapeutic agents (e.g., cytotoxins, nuclides, etc.), and the like. Immunoglobulins or fragments thereof that are linked to such moieties are termed "conjugates." One conjugate of particular interest is comprised of an antibody or fragment thereof linked to a cytotoxin such as, but not limited to, the A-chain (or fragment of A-chain) of ricin, diphtheria or similar toxins. See U.S. Pat. No. 4,590,071 and references cited therein which is incorporated herein by reference.

Chemical modification of the immunoglobulin may involve one or more steps. For example, it may be desirable to provide the immunoglobulin with one or more linking groups prior to final assembly of a conjugate. Examples of linking groups include disulfide addition to amino groups utilizing a heterobifunctional linker such as N-succinimidyl 3-(2-pyridyldithio)propionate (SPDP), or the like, or include carboxyl group-sulfhydryl addition utilizing carbodiimide and the linker mercaptoethylamine (MEA) or the like. Since standard chemical procedures are well known in the art, almost any means of chemically modifying proteins can be used to derivatize immunoglobulins.

Covalent attachment of moieties to the immunoglobulin molecule may be accomplished by reaction of amino acid residues of the amino groups of lysine, the free carboxylic acid groups of glutamic and aspartic acid, the sulfhydryl groups of cysteine or by sulfhydryl group linkers and the various side chains of the aromatic amino acids, as described in EPO Publication No. 0088695, the disclosures of which are hereby incorporated by reference. These procedures often result in the loss of immunoglobulin molecule binding activity due to non-specific binding of the moiety of interest in the antigen binding region of the molecule. The immunoglobulin molecules, that have lost binding activity, are removed from those that have retained binding activity, by binding the modified first immunoglobulin to the second immunoglobulin coupled to a solid support.

The following are offered by way of illustration and not limitation.

EXPERIMENTAL

I. Preparation and Selection of Anti-idiotype Monoclonal Antibody (MoAb) to XMMME-001 MoAbs

Strain C57 mice were immunized with a monoclonal anti-melanoma antibody designated XMMME-001, American Type Culture Collection (A.T.C.C.) Accession No. HB8759, deposited March 26, 1985. The primary immunization was administered in complete Freund's adjuvant (200 μg/mouse) at multiple sites (including footpads). One week later, the secondary immunization (200 μg/mouse) was administered in incomplete Freund's adjuvant, with three subsequent injections in PBS (400 μg/ mouse) given a week apart, each administered in phosphate buffered saline (PBS) at multiple sites including footpads. The final immunization was given several days before fusion and the mice were given 400 μg of XMMME-001 antibody at multiple sites.

Spleen cells were removed and fused with the cell line SP2/O-Ag14, A.T.C.C. Accession No. CRL1581. Cells were plated out in 96 wells in hypoxanthine aminopterin thymidine (HAT) selective media and screened for activity in EIA using the F(ab')$_2$ region of the XMMME001 antibody. Several positive wells were cloned and retested against XMMME-001 antibody or XMMME-003 antibody, A.T.C.C. Accession No. HB9159, deposited July 3, 1986, (control monoclonal anti-melanoma antibody which binds a different epitape than that bound by XMMME-001) for activity. Positive wells were also screened for their reactivity in varied environments. In this example, approximately 0.33 μg of XMMME-001-F(ab')₂ was dotted onto nitrocellulose at the 12:00 position in each of the 96 sites corresponding to 96 wells of a Hybri-dot manifold. XMMME-001 antibody, 0.6 μg, was dotted at (contacted with) the 7:00 position and XMMME-003 antibody, 0.7 μg, at the 5 00 position. Supernatant (diluted 1:2 in tris-buffered saline (TBS)) from antiidiotype XMMME-IO1 hybridoma cultures, A.T.C.C. Accession No. HB 9160, deposited July 3, 1986, was then placed in the manifold and allowed to react with the idiotype. The nitrocellulose was then incubated with either control buffer (pH 7.2) or varying acid pH buffers (pH 3, 4, 5, or 6) to determine the dissociating second environment. Following incubation, goat antimouse IgG₁ preabsorbed with IgM and IgG₂ₐ was added to each of the wells, followed by rabbit anti-goat antiserum labeled with peroxidase.

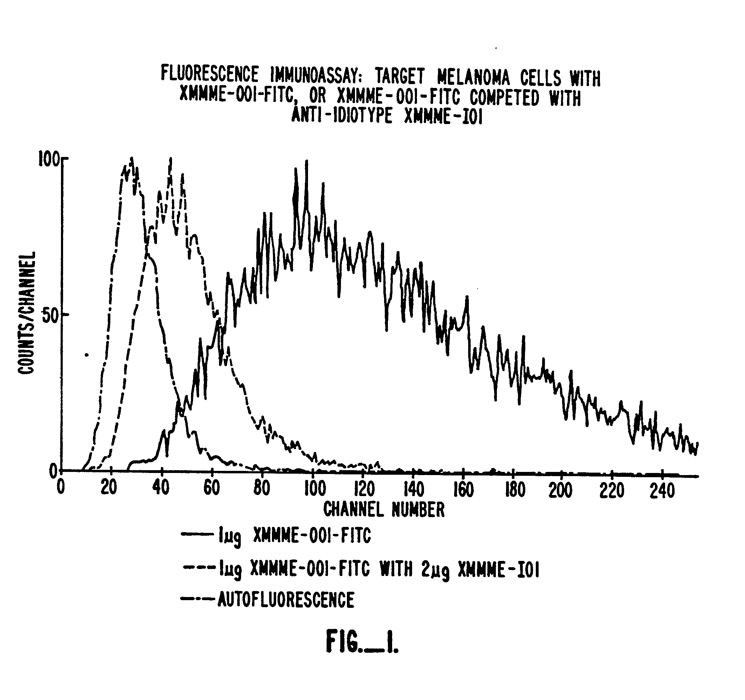

The enzyme substrate 4-Chloronapthol and hydrogen peroxide were then added. A blue dot appearing in any of the wells at the 12:00 and 7:00 but not at the 5:00 position suggested the presence of the anti-idiotype antibody. The clone line XMMME-IO1 of the IgG₁ subclass was found to react only with the XMMME-001 idiotype and the interaction was dissociated at pH 5. XMMME-IO1 antibody also inhibited binding of XMMME-001 to a melanoma cell line (the specific target) in Fluorescence Activated Cell Sorter (FACS) analysis, confirming anti-idiotype activity. (See Fig. 1).

## II. Coupling Procedure: Anti-idiotope on to Solid Phase

Anti-id XMMME-IO1 antibody (4-20 mg/ml in 0.1M sodium bicarbonate with 0.5M NaCl, pH 7.0) was added to activated CH-Sepharose-4B beads (Pharmacia, activated with N-hydroxysuccinimide ester) at 40 to 130 nmole antibody/ml beads. The beads were rocked at 0°C for one minute. The reaction was terminated with the addition of ice cold 1M ethanolamine in 0.5 M NaCl, pH 8.0. The beads were transferred to a column and washed with 15 volumes of ice cold ethanolamine followed by room temperature ethanolamine for 60 minutes, in order to block unreacted groups on the beads. The beads were acid-base washed (five cycles) and finally equilibrated with PBS buffer, pH 8.0 (0.15 M NaCl, 10mM sodium phosphate buffer).

## III. Purification of Monoclonal Antibody (MoAb)

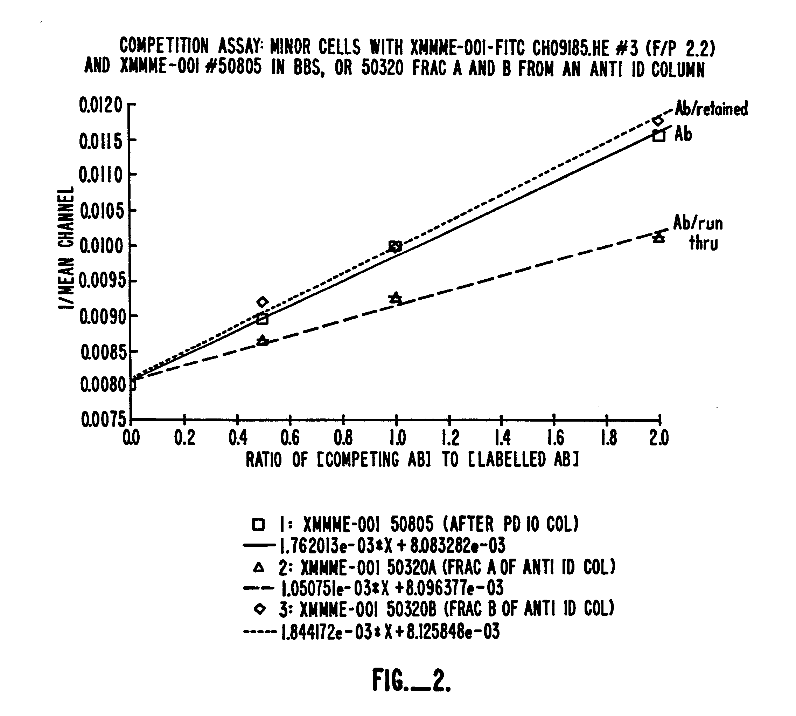

The column, a 5 ml syringe with a bed volume of prepared (anti-idiotope antibody XMMME-IO1 coupled) beads ranging from 2.4-3.8 ml, was used to purify the intact XMMME-001 monoclonal antibody. It was determined that this column had a binding capacity of 0.58 mg XMMME-001 bound per ml of beads. The column was used at 80-90% of binding capacity. The pool of XMMME-001 MoAb that did not bind to the column represented 17% of the starting material and had about half the binding activity of an equivalent amount of starting material by FACS analysis. The bound XMMME-001 MoAb that was pH stripped from the column represented about 74% of the starting material and had about a 5% increase in binding activity by FACS analysis. When a solution of control antibody, XMMME-003 antibody, was passed over the column, only 10% was retained. (See Fig. 2).

This FACS analysis uses an equilibrium competition assay by which relative binding constants can be quantified. Various amounts of the test preparation were mixed with a standard preparation of XMMME-001 MoAb which was labeled with fluorescein isothiocyanate (FITC). Because this assay is performed under equilibrium conditions, relative binding constants can be quantified. A comparison of the test preparation with the starting preparation can be made by determining the ratio of labeled MoAb to competing (test) preparation which produces a 50% decrease in fluorescence intensity. In this case. the starting preparation of XMMME-001 MoAb is compared to the two fractions (bound and unbound) eluted from the column.

## IV. Purification of Immunotoxin

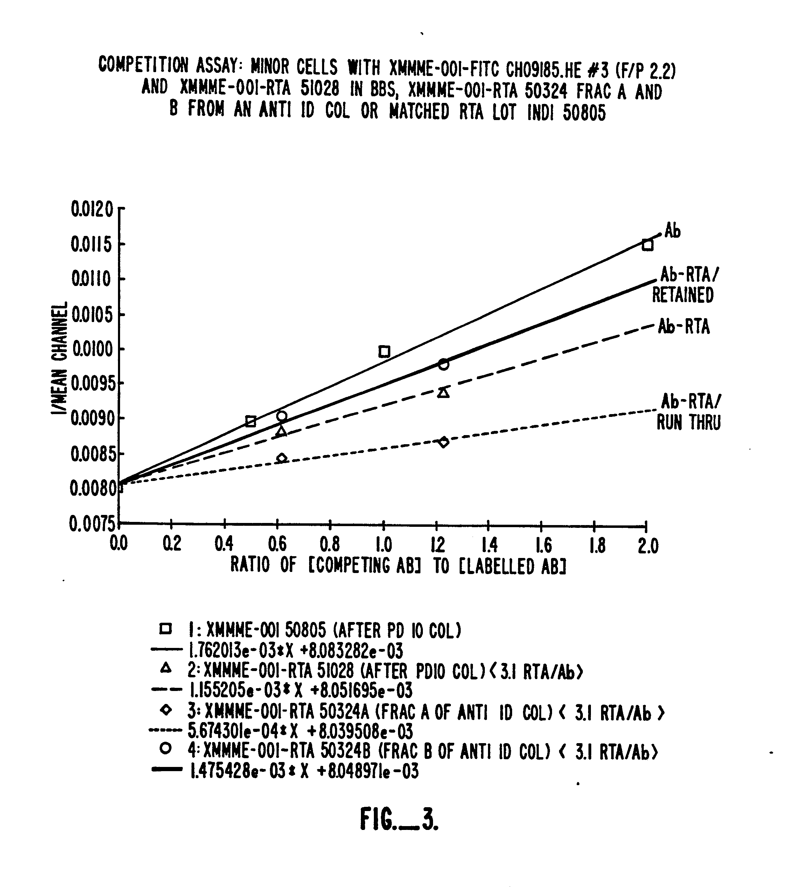

The column, a 10 ml syringe with a bed volume of beads ranging from 6-8 ml, was used to purify immunotoxin (XMMME-001 conjugated to ricin toxin A-Chain [XMMME-001-RTA]. For the conjugation procedure see U.S. Pat. No. 4,590,071, the disclosures of which are hereby incorporated by reference). It was determined that this column had a binding capacity of 0.70 mg of XMMME-001-RTA bound per ml of beads. The column was used at 80-90% of binding capacity, i.e., approximately 0.56 mg of immunotoxin per ml of beads was loaded onto the column. The pool of immunotoxin that did not bind to the column represented between 13-22% of the starting material and had about half the binding activity of the starting material by FACS analysis. The immunotoxin that was pH stripped from the column represented about 50-60% of the starting material and had 24-31% increased binding activity by FACS analysis. (See Fig. 3). FACS analysis of the immunotoxin was the same procedure as already described for the purification of MoAb.

Although the foregoing invention has been described in some detail by way of illustration and example for purposes of clarity and understanding, it will be obvious that certain changes and modifications may be practiced within the scope of the appended claims.

## PREFERRED EMBODIMENTS

1. A purification method for isolating a first immunoglobulin, said method comprising:

(a) coupling to a support a second immunoglobulin which binds to said first immunoglobulin at an idiotopic site under a first environment and dissociates under a second environment wherein said second environment does not alter the binding characteristics of said first and said second immunoglobulins;

(b) contacting in an aqueous medium a sample containing said first immunoglobulin with said support coupled second immunoglobulin under said first environment, thereby forming a complex between said first and said second immunoglobulins;

(c) separating said complex from any non-complexed components in said sample;

(d) dissociating said complex under said second environment; and

(e) collecting said first immunoglobulin in a purified form.

2. A purification method for isolating a first immunoglobulin, said method comprising:

(a) producing a plurality of hybridomas, each of which secretes immunoglobulins specific for an idiotype region of said first immunoglobulin;

(b) selecting from among said hybridomas a hybridoma secreting a second immunoglobulin which binds to said first immunoglobulin at an idiotopic site in said idiotype region under a first environment and dissociates from said first immunoglobulin under a second environment wherein said second environment does not alter the binding characteristics of said first and said second immunoglobulins;

(c) coupling said second immunoglobulin to a support;

(d) contacting in an aqueous medium a sample containing said first immunoglobulin with said support coupled second immunoglobulin under said first environment, thereby forming a complex between said first and said second immunoglobulins;

(e) separating said complex from any non-complexed components in said sample;

(f) dissociating said complex under said second environment; and

(g) collecting said first immunoglobulin in a purified form.

3. A method according to Claim 2, wherein the selecting of said second immunoglobulin secreting hybridoma further comprises:

(a) dotting said first immunoglobulin onto a nitrocellulose support at a plurality of sites;

(b) contacting said second immunoglobulin with said first immunoglobulin dotted sites to form a plurality of first immunoglobulin-second immunoglobulin complexes under said first environment;

(c) varying among said sites said first environment to produce different environments; and

(d) determining said second environment under which said complex dissociates without significantly altering the antigen binding characteristics of either said first or second antibodies.

4. A method according to either Claim 2 or 3, wherein a difference between said first environment and said second environment is a change in pH.

5. A method according to Claim 4, wherein the pH of said first environment is between about 6 and about 10 and the pH of said second environment is between about 3 and about 7.

6. A method according to Claim 4, wherein the pH of said first environment is between about 6.5 and about 8.5 and the pH of the second environment is between about 4 and about 6.

7. A method according to Claim 4, wherein the pH of said first environment is about 8 to 8.5 and the pH of said second environment is about 4.5 to 5.

8. A method according to either Claims 2 or 3, wherein a difference between said first environment and said second environment is a change in ionic strength.

9. A method according to Claim 4, wherein a further difference between said first environment and said second environment is a change in ionic strength.

10. A method according to Claim 2, wherein said support is a solid support.

11. A method according to Claim 10, wherein said solid support is selected from a group consisting of nitrocellulose, cellulose, agarose, polystyrene, dextran, polymer coated magnetic metals, glass, nylon, silica gel, polyacrylamide, and polymethyl methacrylate.

12. A method according to Claim 2, wherein said first immunoglobulin is a monoclonal anti-melanoma antibody designated XMMME-001, produced from hybridoma A.T.C.C. No. HB8759.

13. A method according to Claim 2, wherein said second immunoglobulin is secreted by a hybridoma designated XMMME-IO1, A.T.C.C. No. HB-9160.

14. A method according to Claim 2, wherein said first immunoglobulin is chemically modified.

15. A method according to Claim 14, wherein said first immunoglobulin is radiolabeled.

16. A method according to Claim 14, wherein said first immunoglobulin is fluorescenated.

17. A method according to Claim 14, wherein said chemically modified first immunoglobulin is an immunotoxin.

18. A method according to Claim 17, wherein said immunotoxin contains at least one A-chain (or fragment of A-chain) from ricin.

19. A monoclonal antibody having a high affinity for an idiotype region of an immunoglobulin of interest in a first environment and a low affinity for said idiotype region in a second environment, wherein neither environment significantly alters the antigen binding characteristics of either said monoclonal antibody or said immunoglobulin of interest.

20. A hybridoma designated XMMME-IO1 having A.T.C.C. Accession No. HB9160.

21. In a purification method for isolating a first immunoglobulin from an aqueous sample containing said first immunoglobulin, which comprises producing and selecting a second immunoglobulin directed to bind at an antigenic site on said first immunoglobulin, coupling said second immunoglobulin to a support, contacting said support coupled second immunoglobulin with said aqueous sample to produce a complex, separating said complex from any non-complexing components in said sample, dissociating said complex, and isolating said first immunoglobulin in purified form, the improvement which comprises:

employing an anti-idiotype second immunoglobulin selected to bind at an antigenic site that is an idiotope in an idiotype region of said first immunoglobulin under a first environment; and

dissociating said complex under a second environment which does not alter the binding characteristics of either said first or said second immunoglobulins.

22. A method according to Claim 21, wherein a difference between said first and second environments is a change in pH.

23. A method according to Claim 21, wherein a difference between said first and second environments is a change in ionic strength.

24. A method according to Claim 22, wherein a further difference between said first and second environments is a change in ionic strength.

25. A method according to Claim 21, wherein said support is a solid support.

26. A method according to Claim 25, wherein said solid support is a particle or container wall.

27. A method according to Claim 25, wherein said solid support is selected from a group consisting of nitrocellulose, cellulose, agarose, polystyrene, dextran, polymer coated magnetic metals, glass, nylon, silica gel, polyacrylamide, and polymethyl methacrylate.

28. A method according to Claim 21, wherein said first immunoglobulin is chemically modified.

29. A method according to Claim 28, wherein said first immunoglobulin is radiolabeled.

30. A method according to Claim 28, wherein said first immunoglobulin is fluorescenated.

31. A method according to Claim 28, wherein said chemically modified first immunoglobulin is an immunotoxin.

32. A method according to Claim 31, wherein said immunotoxin contains at least one A-chain or fragment of A-chain from ricin.

33. A conjugate comprising a support coupled to an anti-idiotype antibody or fragment thereof selected to bind to an antibody of interest or fragment thereof at an antigenic idiotype region of said antibody of interest or fragment thereof under a first environment and to dissociate from said antibody of interest or fragment thereof under a second environment.

34. A conjugate according to Claim 33, wherein said second environment does not significantly alter the antigenic binding characteristics of either said anti-idiotype antibody or said antibody of interest or said fragments thereof.

35. Immunoglobulins produced by hybridoma XMMME-IO1 having A.T.C.C. Accession No. HB9160.

**Claims**

1. A purification method for isolating a first immunoglobulin, said method comprising:

(a) coupling to a support a second immunoglobulin which binds to said first immunoglobulin at an idiotopic site under a first environment and dissociates under a second environment wherein said second environment does not alter the binding characteristics of said first and said second immunoglobulins;

(b) contacting in an aqueous medium a sample containing said first immunoglobulin with said support coupled second immunoglobulin under said first environment, thereby forming a complex between said first and said second immunoglobulins;

(c) separating said complex from any non-complexed components in said sample;

(d) dissociating said complex under said second environment; and

(e) collecting said first immunoglobulin in a purified form.

2. A purification method for isolating a first immunoglobulin. said method comprising:

(a) producing a plurality of hybridomas, each of which secretes immunoglobulins specific for an idiotype region of said first immunoglobulin;

(b) selecting from among said hybridomas a hybridoma secreting a second immunoglobulin which binds to said first immunoglobulin at an idiotopic site in said idiotype region under a first environment and dissociates from said first immunoglobulin under a second environment wherein said second environment does not alter the binding characteristics of said first and said second immunoglobulins;

(c) coupling said second immunoglobulin to a support;

(d) contacting in an aqueous medium a sample containing said first immunoglobulin with said support coupled second immunoglobulin under said first environment, thereby forming a complex between said first and said second immunoglobulins;

(e) separating said complex from any non-complexed components in said sample;

(f) dissociating said complex under said second environment; and

(g) collecting said first immunoglobulin in a purified form.

3. A method according to Claim 2, wherein the selecting of said second immunoglobulin secreting hybridoma further comprises:

(a) dotting said first immunoglobulin onto a nitrocellulose support at a plurality of sites;

(b) contacting said second immunoglobulin with said first immunoglobulin dotted sites to form a plurality of first immunoglobulin-second immunoglobulin complexes under said first environment;

(c) varying among said sites said first environment to produce different environments; and

(d) determining said second environment under which said complex dissociates without significantly altering the antigen binding characteristics of either said first or second antibodies.

4. A method according to Claim 2, wherein said first immunoglobulin is a monoclonal anti-melanoma antibody designated XMMME-001, produced from hybridoma A.T.C.C. No. HB8759.

5. A method according to Claim 2, wherein said second immunoglobulin is secreted by a hybridoma designated XMMME-IO1, A.T.C.C. No. HB-9160.

6. A monoclonal antibody having a high affinity for an idiotype region of an immunoglobulin of interest in a first environment and a low affinity for said idiotype region in a second environment, wherein neither environment significantly alters the antigen binding characteristics of either said monoclonal antibody or said immunoglobulin of interest.

7. A hybridoma designated XMMME-IO1 having A.T.C.C. Accession No. HB9160.

8. In a purification method for isolating a first immunoglobulin from an aqueous sample containing said first immunoglobulin, which comprises producing and selecting a second immunoglobulin directed to bind at an antigenic site on said first immunoglobulin, coupling said second immunoglobulin to a support, contacting said support coupled second immunoglobulin with said aqueous sample to produce a complex, separating said complex from any non-complexing components in said sample, dissociating said complexed, and isolating said first immunoglobulin in purified form, the improvement which comprises:

employing an anti-idiotype second immunoglobulin selected to bind at an antigenic site that is an idiotope in an idiotype region of said first immunoglobulin under a first environment; and

dissociating said complex under a second environment which does not alter the binding characteristics of either said first or said second immunoglobulins.

9. A conjugate comprising a support coupled to an anti-idiotype antibody or fragment thereof selected to bind to an antibody of interest or fragment thereof at an antigenic idiotype region of said antibody of interest or fragment thereof under a first environment and to dissociate from said antibody of interest or fragment thereof under a second environment.

10. Immunoglobulins produced by hybridoma XMMME-IO1 having A.T.C.C. Accession No. HB9160.

EP 0 293 606 A2

FLUORESCENCE IMMUNOASSAY: TARGET MELANOMA CELLS WITH XMMME-OOI-FITC, OR XMMME-OOI-FITC COMPETED WITH ANTI-IDIOTYPE XMMME-IOI

—— Iµg XMMME-OOI-FITC

---Iµg XMMME-OOI-FITC WITH 2µg XMMME-IOI

—·—AUTOFLUORESCENCE

**FIG.—I.**

COMPETITION ASSAY: MINOR CELLS WITH XMMME-001-FITC CH09185.HE #3 (F/P 2.2) AND XMMME-001 #50805 IN BBS, OR 50320 FRAC A AND B FROM AN ANTI ID COLUMN

□ I: XMMME-001 50805 (AFTER PD 10 COL)
—— 1.762013e-03*X + 8.083282e-03
△ 2: XMMME-001 50320A (FRAC A OF ANTI ID COL)
— — 1.050751e-03*X + 8.096377e-03
◇ 3: XMMME-001 50320B (FRAC B OF ANTI ID COL)
----- 1.844172e-03*X + 8.125848e-03

FIG._2.

COMPETITION ASSAY: MINOR CELLS WITH XMMME-OOI-FITC CH09I85.HE #3 (F/P 2.2)
AND XMMME-OOI-RTA 5I028 IN BBS, XMMME-OOI-RTA 50324 FRAC A AND
B FROM AN ANTI ID COL OR MATCHED RTA LOT INDI 50805

□    I: XMMME-OOI 50805 (AFTER PD IO COL)
——  1.762013e-03*X +8.083282e-03
△    2: XMMME-OOI-RTA 5I028 (AFTER PDIO COL) ⟨ 3.1 RTA/Ab⟩
— —  1.155205e-03* X +8.051695e-03
◇    3: XMMME-OOI-RTA 50324A (FRAC A OF ANTI ID COL) ⟨ 3.1 RTA/Ab ⟩
----- 5.674301e-04*X +8.039508e-03
O    4: XMMME-OOI-RTA 50324B (FRAC B OF ANTI ID COL) ⟨ 3.1 RTA/Ab⟩
——  1.475428e-03* X +8.048971e-03

FIG._3.